# EUROPEAN PATENT APPLICATION

(11) **EP 3 323 412 A1**
(43) Date of publication of application: **23.05.2018**
(21) Application number: 16199373.8
(22) Date of filing: 17.11.2016
(51) Int. Cl.: A61K 9/20, A61K 9/48, A61K 31/415

(54) **FORMULATION COMPRISING 4-METHYLPYRAZOLE**

(71) Applicant: Amalicon Holding AG, 6340 Baar (CH)
(72) Inventor: Ahrweiler, Michael, 10557 Berlin (DE); Föger, Florian, 6416 Obsteig (AT); Richter, Wolfgang, 81243 Munich (DE)
(74) Representative: Forstmeyer, Dietmar

(57) **Abstract**

The present invention relates to a formulation comprising 4-methylpyrazole or a physiologically acceptable salt thereof and a polyethylene glycol having a total weight average molecular weight of from 200 to 1000 and/or a Magnesium Alumino-metasilicate and the use thereof for treating or preventing ethanol intolerance or toxic side effects of acetaldehyde accumulation or as an antidote in confirmed or suspected methanol or ethylene glycol poisoning.

## Description

The present invention relates to a formulation comprising 4-methylpyrazole and the use thereof for treating, ameliorating or preventing ethanol intolerance or toxic side effects of acetaldehyde accumulation or as an antidote in confirmed or suspected methanol or ethylene glycol poisoning.

4-Methylpyrazole (Fomepizole or 4-MP or 4-methyl-1*H*-pyrazole) is used as an antidote in confirmed or suspected methanol or ethylene glycol poisoning. Fomepizole is a competitive inhibitor of alcohol dehydrogenase, the enzyme that catalyzes the initial steps in the metabolism of ethylene glycol and methanol to their toxic metabolites. Further, 4-methylpyrazole is effective in treating ethanol intolerance by inhibiting aldehyde accumulation in subjects with reduced or absent aldehyde dehydrogenase 2 (ALDH2) activity who have consumed alcohol.

US 2012/0244217 discloses an orally available formulation of 4-MP that is useful to treat ethanol intolerance. Therein it is further stated that the preparation of solid formulations of 4-MP which remain stable under storage conditions is difficult. The inventors of that patent application produced capsules using PEG formulations having an average molecular weight between 5,000 to about 10,000. They did however not succeed in producing stable formulations using low molecular weight PEG having a molecular weight between 200 and 1,000. It is also taught that tablets may be prepared using standard tabletting procedures. However, no examples are given and it is known in the art that by using the excipients disclosed in US 2012/0244217 for preparing the capsules no tablets can be prepared because of the softness of the materials used. The inventors of the above mentioned patent application disclosed that, in contrast to other excipients, high molecular weight polyethylene glycols are particularly suitable for the formulations disclosed in US 2012/0244217.

The present invention relates to formulations comprising liquid PEGs with an average molecular weight of from 200 to 1,000 which are stable in capsules. The advantages of these new formulations are their ease of preparation, the cheap excipients and their comfort in administration and application. In addition, tablets comprising 4-MP are described.

The present invention provides a formulation comprising:
a) 4-methylpyrazole or a physiologically acceptable salt thereof; and
b)
   b1) a polyethylene glycole having a total weight average molecular weight of from 200 to 1000; and/or
   b2) a magnesium aluminometasilicate.

The present invention moreover relates to the use of the formulations described herein for the treatment of acetaldehyde toxicity resulting from ALDH2 deficiency.

Further, the formulations of the present invention can be used as an antidote in confirmed or suspected methanol, ethanol or ethylene glycol poisoning.

Preferably, the formulations of the present invention are provided as a unit dosage. Further preferably but not limited to these, the unit dosage is in the form of a tablet, a sublingal tablet, a sachet, a softgel capsule or a capsule.

Further preferably, the formulation of the present invention comprises from about 0.5 mg 4-MP to about 600 mg 4-MP. More preferably, the formulation of the present invention comprises from 0.5 mg 4-MP to 5.0 mg 4-MP; from 5.0 mg 4-MP to 50 mg 4-MP; from 10 mg 4-MP to about 50 mg 4-MP; from 40 mg 4-MP to 400 mg 4-MP; from 50 mg 4-MP to about 500 mg 4-MP; or from 20 mg 4-MP to about 600 mg 4-MP.

Preferably, the formulation of the present invention comprises from about 5% by weight to about 60% by weight of 4-MP, or a physiologically acceptable salt thereof based on the total formulation. Further preferably, the formulation of the present invention comprises from 10% by weight to 50% by weight of 4-MP, or a physiologically acceptable salt thereof. Especially preferably, the formulation of the present invention comprises from 20% by weight to 40% by weight of 4-MP, or a physiologically acceptable salt thereof.

Moreover preferably, the formulation is physically and/or chemically stable for 8 months or more at room temperature and at a relative humidity of up to about 60% ± 5%. For example, in certain embodiments, the formulation is provided as a capsule or a tablet; preferably it displays no deformity, breakage, or leakage of contents, and retention of at least about 90% content of active pharmaceutical unit (i.e., 4-MP) when stored at room temperature and at a relative humidity of up to about 60% ± 5% for 8 months or more.

The polyethylene glycol used in the present invention has a total weight average molecular weight of from 200 to 1000; preferably from about 300 to about 800; especially preferably from about 300 to about 600.

In certain embodiments, the polyethylene glycol is selected from the group consisting of Polyethylene Glycol 300 (PEG 300), Polyethylene Glycol 400 (PEG 400), Polyethylene Glycol 600 (PEG 600), Polyethylene Glycol 800 (PEG 800), and a combination thereof. Preferably, the polyethylene glycol is selected from PEG 300 and PEG 400.

In one embodiment, the polyethylene glycol is selected from the group of Super Refined^{®} PEG 300, Super Refined™ PEG 400, Super Refined™ PEG 600.

In one embodiment, the 4-MP formulations comprising a polyethylene glycol having a total average molecular weight of from 200 to 1000 are liquid at room temperature.

Preferably, the Magnesium Alumino-metasilicate is represented by the empirical formula Al₂O₃·MgO·1.7SiO₂·xH₂O wherein x is preferably from 0 to 10. Further preferred is a magnesium aluminometasilicate having the CAS Number 12511-31-8. Especially preferably, the Magnesium Alumino-metasilicate ist Neusilin^{®}. Neusilin^{®} is a synthetic amorphous form of Magnesium Alumino-metasilicate. It is a multifunctional excipient that can be used in both direct compression and wet granulation of solid dosage forms.

According to a further preferred embodiment, the formulation of the present invention further comprises a calcium hydrogen phosphate or dibasic calcium phosphate anhydrous (DCPA), CaHPO₄. A preferred calcium hydrogen phosphate is that sold under the trademark FujiCalin by Fuji Chemical Industries. A preferred calcium hydrogen phosphate is described in US 5,486,365 which is incorporated by reference.

According to a further preferred embodiment, the formulation of the present invention comprises a coating of an anionic copolymer of methacrylic acid and methyl methacrylate. Especially preferably, the formulation of the present invention comprises a coating of Eudragit^{®}.

The formulations of the present invention may further comprise one or more additional excipients. In certain embodiments, the one or more additional excipients may be selected from the group consisting of binders, fillers, diluents, glidants, lubricants, surfactants, emulsifying agents, disintegrants, coatings, flavors, colors, sweetening agents, preservatives, sorbents, and any other additive known to one of skill in the art.

Suitable binders may include, but are not limited to, hydroxypropyl cellulose, hydroxypropyl methyl cellulose, microcrystalline cellulose, polyvinylpyrrolidone, polyvinylpolypyrrolidone, polyethylene glycol, gum tragacanth, gelatin, and the like.

Suitable fillers and/or diluents may include, but are not limited to, lactose, glucose, sucrose, mannitol, sorbitol, isomalt (e.g. isomalt 721), calcium carbonate, calcium sulfate, calcium phosphate, hydroxypropyl cellulose, microcrystalline cellulose, silicified microcrystalline cellulose, water, ethanol, polyethylene glycol, propylene glycol, glycerol, starch, polyvinylpyrrolidone, magnesium stearate, magnesium metasilicate aluminate, and the like.

Suitable glidants may include, but are not limited to, silicon dioxide, colloidal silicon dioxide, talc, magnesium carbonate, and the like.

Suitable lubricants may include, but are not limited to, stearic acid; stearic acid metal salts such as calcium stearate or magnesium stearate; talc; colloidal silica; waxes such as beeswax; sulfates such as sodium sul ate; glycol; lauryl sulfates such as sodium lauryl sulfate or magnesium lauryl sulfate; silicates such as silicic anhydride or silicate hydrate or products like Aerosil^{®} or Aeroperl^{®}; and the like.

Suitable surfactants may include, but are not limited to, anionic surfactants such as sodium dodecyl sulfate; cationic surfactants such as hexadecyl trimethyl ammonium bromide; amphoteric surfactants such as cocamidopropyl betaine; nonionic surfactants such as propylene glycol monocaprylate (Capryol 90^{®}) or diethylene glycol monoethyl ether (Transcutol^{®}); and the like.

Suitable disintegrants may include, but are not limited to, cellulose derivatives such as low-substituted hydroxypropyl cellulose, carboxymethyl cellulose, calcium carboxymethyl cellulose or internally crosslinked sodium carboxymethyl cellulose, microcrystalline cellulose (MCC); cross-linked polyvinylpyrrolidone (e.g. crospovidone); and chemically modified starches/celluloses such as carboxymethyl starch, sodium carboxymethyl starch, sodium starch glycolate, pregelatinised starch or croscarmellose sodium; and the like. Further preferred disintegrants are Croscarmellose sodium (Ac-Di-Sol), Crosslink polyvinylpyrrolidone (Kollidon-CL) and Carmellose calcium (ECG-505).

Suitable coatings may include, but are not limited to, hydroxy propylmethylcellulose (HPMC), gelatin, enteric coating polymers such as Eudragit^{®} and the like.

Coatings may be applied using an appropriate technology such as airsuspension using an enteric coating polymer such as Eudragit^{®} or hydroxy propylmethylcellulose (HPMC), gelatin, and the like.

Suitable preservatives may include, but are not limited to, cysteine, methionine, citric acid, sodium citrate, methyl paraben, propyl paraben, and the like.

Tablets may be prepared using any tableting technique known to one of skill in the art. For example, tablets may be prepared by a direct compression method, wherein the bulk blend is transferred directly to a compression machine for pressing into a tablet. Other conventional methods such as wet granulation or dry granulation may also be used.

The formulation of the present invention is especially suitable for the preparation of mini tablets (e.g. having a diameter of about 1 mm to about 4 mm) which can be filled into capsules. Such a preparation has the advantage that leakage of the 4-MP can be prevented. Further it provides for a simple individual dosage for patients.

If the formulation of the present invention is in the form of a tablet, it preferably further contains at least one disintegrant.

A capsule may be prepared using any capsule-filling technique known to one of skill in the art. For example, a capsule may be prepared by transferring the bulk blend directly to a capsule filling and sealing machine. The capsule may be comprised of gelatin, plasticized gelatin, hydroxypropylmethylcellulose (HPMC), cellulose, starch or agar, or any other material known to one of skill in the art. Preferred capsules are the gelatin or cellulose capsules sold under the trademark Torpac^{®}. Further preferred capsules are capsules sold under the trademark Licaps^{®} and PEG/gelatine capsules. Morover preferred Capsules are soft capsules sold under the trademark Vegicaps^{®} and Polyvinyl alcohol (PVA) copolymer capsules.

Further preferred capsules are Coni-Snap^{®} Sigma Series Hard Gelatin Capsules, *Quali*-*G™* hard gelatine capsules or soft gelatine capsules.

In certain embodiments, the capsule comprises gelatin. In certain embodiments, the capsule comprises soft gelatin. In certain embodiments, the capsule comprises hard gelatin.

One or more plasticizer(s) may be added to the capsule material to increase the flexibility and strength and may be selected from glycerin, propylene glycol, polyethylene glycol, triethyl citrate, acetyl triethyl citrate, diethyl phthalate, dibutyl phthalate, dibutyl sebacate, or mixtures thereof, or any other material or mixture known to one of skill in the art. The plasticizer may be present in an amount ranging from 0.1% to 30% by weight of the capsule.

The capsules may be sized to hold the desired amount of the formulation, typically up to about 0.50 ml of the formulation. Preferably, the size of any particular capsule described herein will correspond to a conventional capsule size, e.g. Size Nos. 00, 0, 1, 2, 3, 4, 5, and the like.

The tablets and capsules may also be coated with an enteric coating, alone or in addition to another coating. Materials suitable for use in the enteric coating include hydroxypropyl methylcellulose, hydroxyethyl cellulose, hydroxypropyl cellulose, methylcellulose, ethylcellulose, acrylic acid methacrylic acid ester copolymer, or a mixture thereof. Additional materials suitable for use in the enteric coating include phthalates including hydroxypropyl methylcellulose phthalate, hydroxyethyl cellulose phthalate, hydroxypropyl cellulose phthalate, methylcellulose phthalate, ethylcellulose phthalate, and cellulose acetate phthalate.

The tablets and capsules may additionally be coated with a controlled release coating, which is compatible with the other components of the enteric coating. The controlled release coating may comprise a hydrophobic controlled release material selected from an alkylcellulose, an acrylic polymer, or mixtures thereof. The controlled release coatings may also include a plasticizer such as those described herein.

The formulations provided herein can be administered according to any technique known to one of skill in the art. In preferable embodiments, the subject can self-administer the formulation to himself or herself. In preferable embodiments, the formulation can be administered orally. When orally administered, the formulation is preferably in a solid form. When orally administered, the formulations can also be in a unit dosage form, for example, as in a tablet, capsule and the like, as provided herein.

The formulation can be administered alone or in combination with other substances or active agents. In some embodiments, a formulation and other ingredients, as described below, is administered.

Preferably, the amount of 4-MP administered in the formulation is from about 0.05 mg/kg body weight to about 30.0 mg/kg body weight (e.g. from about 0.05 mg/kg body weight to about 12.0 mg/kg body weight); further preferably from about 0.5 mg/kg body weight to about 25.0 mg/kg body weight; especially preferably from about 5 mg/kg body weight to about 20.0 mg/kg body weight or from about 0.5 mg/kg body weight to about 6.0 mg/kg body weight.

The formulation provided herein, when administered to a subject, is preferably effective to reduce or inhibit the ethanol-oxidizing activity of alcohol dehydrogenase (ADH) in the subject.

In certain embodiments, the formulations provided herein are suitable for administration to subjects wishing to reduce or ameliorate a symptom of ethanol intolerance or acetaldehyde accumulation. In certain embodiments, the subject has reduced or absent aldehyde dehydrogenase subtype 2 (ALDH2) activity. In certain embodiments, the subject is a human. Especially preferably, the formulations of the present invention are suitable for oral administration in a human.

The formulations of the present invention are further suitable for the prolonged treatment (maintenance therapy) of poisoning, e.g. afer a subject has regained consciousness after an intitial iv treatment with 4-MP.

In certain embodiments, the formulation can be administered before the subject has consumed ethanol, it can be administered concurrent with the consumption of ethanol and/or the formulation can be administered to a subject after the subject has consumed ethanol.

In certain aspects, the present invention provides methods for preventing a disease associated with peak levels of acetaldehyde derived from the long term or frequent use of ethanol in a subject with ALDH2 deficieny. In general, diseases associated with high exposure to acetaldehyde through the long term use of ethanol include, for example and without limitation, liver cirrhosis and cancer, for example, hepatocellular carcinoma, mouth cancer, stomach cancer, and esophageal cancer.

In certain preferable embodiments of methods provided for preventing a disease associated with high levles of acealdehyde during the long term use of ethanol in a subject, 4-MP is administered prior to the consumption of ethanol by the subject. In some embodiments, 4-MP can be administered within about two hours before the subject consumes ethanol.

As used herein, "about" indicates a range of +/-10%. For example, "about 10 mg 4-MP" means a range of from 9.0 mg to 11.0 mg 4-MP.

As used herein, "ethanol intolerance," refers to a condition in which a subject experiences a symptom of acetaldehyde accumulation accompanying ethanol consumption. Symptoms of ethanol intolerance, or acetaldehyde accumulation, may include, but are not limited to, flushing, elevated heart rate, palpitations, hypotension, nausea, dizziness, headache, vomiting, diarrhea, upset stomach, ataxia, or confused consciousness. As used herein, "acetaldehyde accumulation" refers to the production of acetaldehyde in a subject that has consumed ethanol.

The term "effective amount" as used herein refers to the amount of 4-MP, or physiologically acceptable salt thereof, that is sufficient to produce a desirable or beneficial effect when contacted, for example, to an alcohol dehydrogenase enzyme, or, as another example, when administered to a subject. In certain embodiments, the "effective amount" is, for example, the amount to prevent, reduce or ameliorate a symptom associated with acetaldehyde accumulation in a subject accompanying ethanol consumption, or to reduce the likelihood or risk, in a subject for a disease or disorder caused by consumption of ethanol.

As used herein, the term "dose" or "dosage" refers to the amount of 4-MP that an subject takes or is administered at one time. As used herein, the term "unit dosage" or "dosage unit" refers to a physically discrete unit, such as a tablet or capsule, suitable as a unitary dosage for a subject.

As defined herein, where the mass of 4-MP is specified, for example, "10 mg 4-MP," that amount refers to the equivalent mass of 4-MP in its free base form.

The term "physiologically acceptable salt" or "acceptable salt form," as used herein, refers to the relatively nontoxic, inorganic and organic acid addition salts of compounds of the invention. Examples of physiologically acceptable salts of 4-methylpyrazole are salts of physiologically acceptable mineral acids like hydrochloric, hydrobromic, sulfuric and phosphoric acid; or salts of organic acids like methanesulfonic, p-toluenesulfonic, lactic, acetic, trifluoroacetic, citric, succinic, fumaric, maleic and salicylic acid.

The term "room temperature," as used herein, refers to 25°C ± 2 °C.

The term "treat", "treating" or "treatment", as used herein, refers to a method of alleviating or abrogating a disorder and/or its attendant symptoms.

A preferred formulation of the present invention comprises:

| | |
|---|---|
| 10 to 60 % by weight | 4-MP |
| 10 to 50 % by weight | Magnesium Alumino-metasilicate |
| 10 to 50 % by weight | of at least one disintegrant |
| 10 to 70 % by weight | of at least one filler. |

### Examples

A. Formulations that are suitable for the preparation of tablets:

### Example 1:

| | |
|---|---|
| 4-MP | 25,00% |
| Neusilin | 25,00% |
| Fujicalin | 22,50% |
| MCC Spray dried | 22,50% |
| Crospovidone | 3,00% |
| Magnesiumstearate | 2,00% |
| Total (m/m) | 100,00% |

### Example 2:

| | |
|---|---|
| 4-MP | 25,00% |
| Neusilin | 15,00% |
| Isomalt 721 | 55,00% |
| Crospovidone | 3,00% |
| Magnesiumstearate | 2,00% |
| Total (m/m) | 100,00% |

### B. Formulations in the form of a capsule

### Example 3:

50 mg 4-MP were mixed with 250 mg of PEG 400 and filled into a Torpac^{®} capsule.

### Example 4:

50 mg 4-MP were mixed with 250 mg of PEG 300 and filled into a Torpac^{®} capsule.

### Example 5:

Storage stability of liquid 4-MP formulations with HPMC or hard gelatine capsules at room temperature in closed 4 ml screw cap bottles.

| **Composition** | **1 week** | **2 month** | **3 month** | **6 month** |
|---|---|---|---|---|
| **100% 4-MP in HPMC capsule** | Capsule completely collapsed | n.a. | n.a. | n.a. |
| **100% 4-MP in Gelatine capsule** | Capsule partially collapsed | n.a. | n.a. | n.a. |
| **25% 4-MP and 75% PEG300 in Gelatine capsule** | Capsule intact | Capsule intact | Capsule intact | Capsule intact |
| **25% 4-MP and 75% PEG400 in Gelatine capsule** | Capsule intact | Capsule intact | Capsule intact | Capsule intact |

The formulations of 4-MP with PEG-300 and PEG-400 show an oral bioavailability of about 110% when compared with an i.v. formulation at a dose of 10mg/kg in PK studies in dogs.

The following tables 1 to 3 show results of pharmacokinetic studies of the formulation of Example 3 (capsule containing 50 mg 4-MP and 250 mg PEG-400; Table 1) in comparison with a reference formulation (capsule containing 50 mg 4-MP and PEG-8000; Table 2) and IV administration of 4-MP. For these pharmacokinetic studies, 4-MP was administered to dogs at 10 mg/kg body weight.

**Table 1: Pharmacokinetic parameters of 4-methylpyrazole following oral administration of the formulation of Example 3 at 10 mg/kg:**

| **parameter** | **Dog no.** | | | **Mean** | **S.D.** | **CV%** |
|---|---|---|---|---|---|---|
| | **1** | **2** | **3** | | | |
| **t_{1/2,β} (h)** | 5.55 | 6.13 | 6.07 | **5.91** | **0.32** | **5.42** |
| **AUC₀₋ₜ (ng*h/mL)** | 104597 | 161294 | 112338 | **126076** | **30744** | **24.4** |
| **AUC_{0-∞} (ng*h/mL)** | 133737 | 211793 | 149256 | **164929** | **41321** | **25.1** |
| **Cₘₐₓ (ng/mL)** | 14439 | 21379 | 19478 | **18432** | **3586** | **19.5** |
| **Tₘₐₓ (h)** | 3.00 | 8.00 | 0.25 | **3.75** | **3.93** | **105** |
| **Tₗₐₛₜ (h)** | 12.0 | 12.0 | 12.0 | **12.0** | 0.00 | **0.00** |
| **% Bioavailability (%F)** | **110** | | | | | |
| Time points considered in t_{1/2, β} calculation | 6 - 12 h | | | | | |

**Table 2: Pharmacokinetic parameters of 4-methylpyrazole following oral administration of the reference formulation at 10 mg/kg.**

| **parameter** | **Dog no.** | | | **Mean** | **S.D.** | **CV%** |
|---|---|---|---|---|---|---|
| | **1** | **2** | **3** | | | |
| **t_{1/2,β} (h)** | 5.10 | 8.97 | 5.26 | **6.44** | **2.19** | **33.9** |
| **AUC₀₋ₜ (ng*h/mL)** | 113928 | 134789 | 120735 | **123151** | **10639** | **8.64** |
| **AUC_{0-∞} (ng*h/mL)** | 143290 | 222508 | 150343 | **172047** | **43842** | **25.5** |
| **Cₘₐₓ (ng/mL)** | 16893 | 20293 | 18356 | **18514** | **1705** | **9.21** |
| **Tₘₐₓ (h)** | 0.75 | 1.00 | 1.00 | **0.92** | **0.14** | **15.7** |
| **Tₗₐₛₜ (h)** | 12.0 | 12.0 | 12.0 | **12.0** | **0.00** | **0.00** |
| **% Bioavailability (%F)** | **115** | | | | | |
| Time points considered in t_{1/2, β} calculation | 6 - 12 h | | | | | |

**Table 3: Pharmacokinetic parameters of 4-methylpyrazole following IV administration at 10 mg/kg**

| **parameter** | **Dog no.** | | | **Mean** | **S.D.** | **CV%** |
|---|---|---|---|---|---|---|
| | **1** | **2** | **3** | | | |
| **t_{1/2,β} (h)** | 9.48 | 10.4 | 4.71 | **8.20** | **3.05** | **37.2** |
| **Cₘₐₓ (ng/mL)** | 22600 | 22536 | 22394 | **22510** | **105** | **0.47** |
| **AUC₀₋ₜ (ng*h/mL)** | 88311 | 111233 | 94094 | **97879** | **11920** | **12.2** |
| **AUC_{0-∞} (ng*h/mL)** | 145010 | 189552 | 114143 | **149568** | **37911** | **25.3** |
| **%Extrapolated AUC** | 39.1 | 41.3 | 17.6 | **32.7** | **13.1** | **40.2** |
| **CL (mL/min/kg)** | 1.15 | 0.88 | 1.46 | **1.16** | **0.29** | **25.0** |
| **V_{d} (L/kg)** | 0.94 | 0.79 | 0.60 | **0.78** | **0.17** | **22.4** |
| **V_{dss} (L/kg)** | 0.89 | 0.73 | 0.59 | **0.74** | **0.15** | **20.1** |
| Time points considered in t_{1/2, β} calculation | 6 - 12 h | | | | | |

From these data it can be clearly taken that the formulation of the present invention shows a lower t_{1/2,ß} when compared with the i.v. administration using the same dose of 10mg/kg and also when compared with the reference formulation comprising PEG 8000 as disclosed in US 2012/0244217. Accordingly, the formulation of the present invention has a better efficacy in inhibiting the negative impact of acetaldehyde formed over the time after the consumption of alcohol.

The formulation of the present invention also exhibits a longer Tₘₐₓ which is advantageous for the inhibitory effect of 4-MP against ADH.

## Claims

1. A formulation comprising:
a) 4-methylpyrazole or a physiologically acceptable salt thereof; and
b) b1) a polyethylene glycole having a total weight average molecular weight of from 200 to 1000; and/or b2) a Magnesium Alumino-metasilicate.

2. The formulation according to claim 1, comprising:
a) 4-methylpyrazole or a physiologically acceptable salt thereof; and
b) a polyethylene glycole having a total weight average molecular weight of from 200 to 1000.

3. The formulation according to claim 1, comprising:
a) 4-methylpyrazole or a physiologically acceptable salt thereof; and
b) a Magnesium Alumino-metasilicate.

4. The formulation according to claim 1 or 2, wherein the polyethylene glycole has a total weight average molecular weight of from 200 to 500; especially wherein the polyethylene glycole is PEG 300 or PEG 400.

5. The formulation according to any one of claims 1, 2 or 4 which is in the form of a capsule.

6. The formulation of claim 5 wherein the capsule comprises gelatin or cellulose.

7. The formulation according to claim 1 or 3, wherein the Magnesium Alumino-metasilicate is Neusilin^{®}.

8. The formulation according to any one of claims 1, 3 and 7 which is in the form of a tablet.

9. The formulation according to claim 8 further containing at least one disintegrant.

10. The formulation according to any one of claims 1, 3, 7, 8 and 9 which is in the form of a tablet having a diameter of about 1 mm to about 4 mm.

11. The formulation according to any one of the preceding claims comprising from 10 mg to 500 mg of 4-methylpyrazole.

12. The formulation according to any one of the preceding claims comprising from about 5% by weight to about 60% by weight of 4- methylpyrazole, or a physiologically acceptable salt thereof.

13. The formulation according to any one of the preceding claims further comprising one or more additional excipients.

14. The formulation according to any one of the preceding claims wherein the formulation is suitable for oral administration in a human.

15. The formulation according to any one of the preceding claims for use in preventing, ameliorating or treating a symptom of ethanol intolerance or for use as an antidote in confirmed or suspected methanol or ethylene glycol poisoning.
